Europäisches Patentamt

European Patent Office

Office européen des brevets

(19)

(11) Numéro de publication: **0 112 748**
**A1**

## (12) DEMANDE DE BREVET EUROPEEN

(21) Numéro de dépôt: 83402299.8

(22) Date de dépôt: 29.11.83

(51) Int. Cl.³: **A 61 K 9/00,** A 61 K 9/50, **A 61 K 33/42**

(30) Priorité: 01.12.82 FR 8220123

(43) Date de publication de la demande: 04.07.84
Bulletin 84/27

(84) Etats contractants désignés: **AT BE CH DE GB IT LI LU NL SE**

(71) Demandeur: **GRIMBERG, Georges Serge, 123 rue de l'Université, F-75007 Paris (FR)**

(72) Inventeur: **GRIMBERG, Georges Serge, 123 rue de l'Université, F-75007 Paris (FR)**

(74) Mandataire: **Madeuf, René Louis et al, Cabinet Madeuf 3, Avenue Bugeaud, F-75116 Paris (FR)**

(54) Composition pharmaceutique nouvelle à base de phosphate d'alumine.

(57) La composition pharmaceutique à base de phosphate d'alumine est caractérisée en ce que les particules de mucilage ou les particules équivalentes sont enrobées dans au moins une couche constituée par au moins un composant inerte vis-à-vis d'au moins un produit de la phase continue puis au moins un composant actif vis-à-vis d'au moins un produit de la phase continue sert au moins à enrober les particules de mucilage ou les particules équivalentes susmentionnées.

EP 0 112 748 A1

ACTORUM AG

Composition pharmaceutique nouvelle à base de
phosphate d'alumine.

La présente invention a pour objet une nouvelle composition pharmaceutique à base de phosphate d'alumine
destinée à être dispersée dans une phase continue, cette
nouvelle composition pouvant, par des formes diverses ou
des adjonctions, présenter toujours un haut pouvoir de
dispersion car les médicaments connus, à base de phosphate d'alumine, qui ont comme propriété d'être anti-
acide, protecteur de la muqueuse oeso-gastro-duodénale,
ne permettent pas d'obtenir un revêtement protecteur
complet de la muqueuse et ne donnent pas entière
satisfaction.

Les présentations actuelles des compositions à base de
phosphate d'alumine sont sous des formes différentes,
par exemple en suspension, en granulés, en comprimés, en
poudre, mais malgré ces différentes présentations on
n'obtient pas le résultat recherché car la phase purement
physique n'est pas toujours obtenue et la dispersion
n'est pas bonne ce qui ne permet pas de bien tapisser les
muqueuses.

C'est pourquoi, la présente invention tend à remédier aux
inconvénients ci-dessus en donnant une présentation du
phosphate d'alumine sous une forme pulvérulente destinée
à être dispersée dans une phase continue et notamment
dans une phase aqueuse, ce qui permet non seulement
d'obtenir une présentation pour enfants dont la répartition et l'absorption sont plus uniformes, mais également
une facilité d'utilisation accrue sans longue préparation
par des adultes.

Pour obtenir ce résultat on procède à une dispersion de
la composition de phosphate d'alumine conforme à l'invention présentée sous différentes formes, cette dispersion étant effectuée de façon extemporanée.

Dans ce qui suit, on désigne par dispersion tous les corps composés d'au moins deux phases pharmaceutiquement utilisables, l'une des phases étant continue tandis que l'autre phase est discontinue et est répartie dans la première aussi uniformément que possible. Dans la présente invention, elle concerne des produits de base qui sont constitués par des particules de mucilage ou des particules équivalentes dans lesquelles le phosphate d'alumine mélangé et parfois associé à ces particules se trouve au moment de cette dispersion noyé dans la masse en cours de gonflement dès que les particules de mucilage ou les particules équivalentes se trouvent en présence d'une solution aqueuse.

Conformément à l'invention, les particules de mucilage ou les particules équivalentes sont enrobées dans au moins une couche constituée par au moins un composant inerte vis-à-vis d'au moins un produit de la phase continue puis au moins un composant actif vis-à-vis d'au moins un produit de la phase continue sert au moins à enrober les particules de mucilage ou les particules équivalentes susmentionnées.

Suivant une autre caractéristique de l'invention, lorsque la composition thérapeutique nouvelle constituée d'un mucilage enrobé et d'au moins un additif complémentaire est mise dans une phase aqueuse ou une autre phase continue, on crée une dispersion au sein de laquelle les particules de phosphate d'alumine sont noyés entre les particules de mucilage de sorte qu'au moment de l'ingestion le phosphate d'alumine se trouve très largement dispersé et en une manière uniforme.

0112748

3

Diverses autres caractéristiques de l'invention sont représentées à titre d'exemples non limitatifs.

On donne ci-après plusieurs exemples de la composition pharmaceutique nouvelle à partir du phosphate d'alumine et d'un ou plusieurs composants actifs pouvant également renfermer des gélifiants, des édulcorants et des aromatisants.

EXEMPLE 1

Composition pharmaceutique ingérable :

| | | |
|---|---|---|
| - Phosphate d'alumine | 1.000 | g |
| - Gomme de Guar en poudre | 2.478 | g |
| - Huile de silicone | 12,5 | g |
| - Mono-oléate de sorbitane | 6,44 | g |
| - Polysorbate 80 | 3,22 | g |
| - Silice micronisée | 50,00 | g |
| - Sucre | 6.000 | g |
| - Saccharinate de sodium | 10 | g |
| - Cyclamate de sodium | 40 | g |
| - Noix de coco (Arôme en poudre) | 200 | g |

Lorsque le patient mélange le produit ainsi obtenu dans une phase aqueuse, la gomme de Guar se disperse puis se met à gonfler et au moment du gonflement emprisonne le phosphate d'alumine. Ce phénomène se poursuit in vivo.

On remarque que, dans cet exemple, la gomme de Guar peut être remplacée notamment par des alginates, de la gélatine, des gommes ou des celluloses.

Mais il est remarquable que le produit obtenu soit dans tous les cas de belle apparence, fabriqué rapidement et sans irrégularités. Il peut être aisément conditionné et conservé en récipients, en vue de la dispersion lors de

4

l'utilisation ou transformé sous toute forme galénique convenable telle que comprimés, sachets, etc.

Dans tous les cas, si on disperse la présente composition en milieu liquide, de l'eau par exemple, quelles que soient les proportions entre le produit et l'eau, la répartition sera obtenue rapidement à froid, de façon homogène, sans appareillage spécial. La consistance du gel obtenu variera pour un produit donné, d'une part, avec le temps, d'autre part, avec la quantité relative de poudre et d'eau.

Il suffit pour cela d'opérer le mélange progressivement et de remuer ensuite ce mélange à l'aide d'une cuiller ou de la mesurette, qui a servi au dosage, jusqu'à un premier degré d'épaississement du gel.

EXEMPLE 2

Une composition pharmaceutique est pratiquement identique tant dans sa composition qualitative que dans sa composition quantitative mais, on remplace la gomme de Guar en poudre par des particules de mucilage.

Dans la majorité des cas, il est ajouté à la composition, tant dans l'exemple 1 que dans l'exemple 2, un produit tensio-actif thérapeutiquement acceptable.

Le produit, ainsi obtenu, peut être utilisé comme anti-acide, protecteur de la muqueuse oeso-gastro-duodénale et contre le météorisme.

Il constitue donc une nouvelle forme galénique du phosphate d'alumine, principe actif connu en lui-même.

Les essais classiques réalisés sur ce produit permettent

5

de constater que les propriétés bénéfiques du phosphate d'alumine ne sont pas diminuées et que cette nouvelle forme galénique présente de plus des avantages appréciables sur le plan de l'administration et de la tolérance.

Revendications

1 - Composition pharmaceutique à base de phosphate d'alumine, dans laquelle les particules de mucilage ou les particules équivalentes sont enrobées dans au moins une couche constituée par au moins un composant inerte vis-à-vis d'au moins un produit de la phase continue puis au moins un composant actif vis-à-vis d'au moins un produit de la phase continue sert au moins à enrober les particules de mucilage ou les particules équivalentes susmentionnées; ce qui permet d'obtenir une composition pharmaceutique contenant un mucilage enrobé et au moins un additif complémentaire puis après mise dans une phase aqueuse ou une autre phase continue, on crée ainsi une dispersion au sein de laquelle les particules de phosphate d'alumine sont noyées entre les particules de mucilage de sorte qu'au moment de l'ingestion le phosphate d'alumine se trouve très largement dispersé et d'une manière uniforme; étant noté que la composition contient un ou plusieurs édulcorants et un ou plusieurs aromatisants, caractérisée en ce qu'elle contient au moins un produit actif qui est tensio-actif.

2 - Composition pharmaceutique suivant la revendication 1, caractérisée en ce qu'elle contient :

| | | |
|---|---|---|
| - Phosphate d'alumine | 1 000 | g |
| - Gomme de Guar en poudre | 2 478 | g |
| - Huile de silicone | 12,5 | g |
| - Mono-oléate de sorbitane | 6,44 | g |
| - Polysorbate 80 | 3,22 | g |
| - Silice micronisée | 50,00 | g |
| - Sucre | 6 000 | g |
| - Saccharinate de sodium | 10 | g |
| - Cyclanate de sodium | 40 | g |
| - Noix de coco (Arome en poudre) | 200 | g |

et qu'elle est appliquée en tant qu'anti-acide, protecteur de la muqueuse oeso-gastro-duodénale, et contre le
météorisme.

3 - Composition pharmaceutique suivant l'une des
revendications 1 et 2, caractérisée en ce qu'elle est
utilisable sous différentes formes galéniques.

**Office européen des brevets**

**RAPPORT DE RECHERCHE EUROPEENNE**

EP 83 40 2299

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X,Y | US-A-4 139 612 (J.P. RAUDNITZ)<br>* Colonne 1, lignes 49-63; exemples; revendications * | 1-3 | A 61 K 9/00<br>A 61 K 9/50<br>A 61 K 33/42 |
| Y | FR-A-2 092 107 (W.H. RORER)<br>* Page 1, ligne 40; page 2, ligne 23; exemples 1,4; revendications 1,5 * | 1-3 | |
| Y | FR-A-2 438 431 (G.S. GRIMBERG)<br>* Revendications; page 1, alinéas 1,3,5,6; exemple 2 * | 1-3 | |
| Y | FR-A-2 437 867 (G.S. GRIMBERG)<br>* Page 2, alinéas 2,3; page 5, lignes 5-8; exemple 11; revendications * | 1-3 | **DOMAINES TECHNIQUES RECHERCHES (Int. Cl. ³)**<br><br>A 61 K 9/00<br>A 61 K 33/00 |

Le présent rapport de recherche a été établi pour toutes les revendications

| Lieu de la recherche | Date d'achèvement de la recherche | Examinateur |
|---|---|---|
| LA HAYE | 12-03-1984 | EERTE M.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

&: membre de la même famille, document correspondant

OEB Form 1503. 03 82